# EUROPÄISCHE PATENTSCHRIFT

(11) **EP 1 881 970 B1**
(45) Veröffentlichungstag und Bekanntmachung des Hinweises auf die Patenterteilung: **11.07.2012**
(21) Anmeldenummer: 06724705.6
(22) Anmeldetag: 04.05.2006
(51) Int. Cl.: C07D 317/40

(54) **VERFAHREN ZUR REINIGUNG VON VINYLENCARBONAT**
METHOD OF PURIFYING VINYLENE CARBONATE
PROCEDE POUR NETTOYER DU CARBONATE DE VINYLENE

(30) Priorität: 12.05.2005 DE 102005021966
(43) Veröffentlichungstag der Anmeldung: 30.01.2008
(73) Patentinhaber: Saltigo GmbH, 51369 Leverkusen (DE)
(72) Erfinder: LANGER, Reinhard, 47918 Tönisvorst (DE); WAGNER, Paul, 40597 Düsseldorf (DE); GRZINIA, Heinrich, 41812 Erkelenz (DE)
(74) Vertreter: Pettrich, Klaus-Günter
(86) Internationale Anmeldenummer: PCT/EP2006/004156
(87) Internationale Veröffentlichungsnummer: WO 2006/119910

(56) Entgegenhaltungen:
- US-A1- 2002 107 407
- PATENT ABSTRACTS OF JAPAN Bd. 2003, Nr. 03, 5. Mai 2003 (2003-05-05) & JP 2002 322171 A (MITSUBISHI CHEMICALS CORP), 8. November 2002 (2002-11-08)
- 'Umrechnungstabelle', [Online] Gefunden im Internet: <URL:http://www.hydrogeit.de/einheiten.htm>

## Beschreibung

Die vorliegende Erfindung betrifft die technische Reinigung von Vinylencarbonat (VC).

Vinylencarbonat ist ein wichtiges Zwischenprodukt für die Herstellung von Chemikalien, Pharmaprodukten, Pflanzenschutzmitteln und im Besonderen für Polymere, Lacke und Batterieelektrolyte.

Vinylencarbonat wird nach bekannter Methodik durch Abspalten von Chlorwasserstoff aus Chlorethylenglykolcarbonat (CGC) mittels tertiärer Amine, insbesondere Triethylamin hergestellt.

Das Chlorethylenglykolcarbonat gewinnt man durch radikalische Chlorierung von Ethylenglykolcarbonat mittels Chlor oder Sulfurylchlorid.

Erstmals veröffentlicht wurde diese Synthese 1953 von Newman und Addor (JACS, 1953, S. 1263; JACS 1955, S. 3789).

Ethylenglykolcarbonat (GC) wurde mittels ultraviolettem Licht bei 60-70°C in Substanz photochloriert und das entstandene CGC durch Vakuumdestillation gereinigt.

VC erhielten Newman und Addor durch Eliminierung mittel Triethylamin in siedendem Ether, wobei das Gemisch über Nacht erhitzt wurde.

Die Isolierung erfolgte durch Abfiltrieren des Triethylammoniumchlorides und anschließende Destillation, die mit 59 %iger Ausbeute ein rohes VC lieferte, welches durch weitere Destillation gereinigt werden musste.

JP 2000/026449 beschreibt die Eliminierung in hochsiedenden Lösungsmitteln (Sdp. 170-300°C). Explizit wird mit Triethylamin in Dibutylcarbonat 20 Stunden bei 50°C umgesetzt. Nach dem Abfiltrieren des Ammoniumchlorides und dem Abdestillieren von überschüssigem Triethylamin wird rohes VC durch einfache Destillation isoliert. Um Spuren vom Aminen zu entfernen wird das VC über eine Silicagelsäule gegeben. Abschließend wird eine Feindestillation durchgeführt. Der Chlorgehalt des so gewonnenen VC wird mit 29 ppm angegeben, während Vergleichsproben > 3000 ppm enthalten. Die Ausbeute beträgt 56 %.

DE-A 19 955 944 beansprucht die Eliminierung in GC als Lösungsmittel (Sdp. 243-244°C). CGC wird in GC vorgelegt und in 1,5 Stunden durch Zugabe von Triethylamin bei 60°C umgesetzt. Nach Abdestillieren von überschüssigem Triethylamin bei 40°C und Verdampfen über einen Dünnschichter bei 100°C wird mit 73 %iger Ausbeute ein farbloses Gemisch aus VC und GC gewonnen. Über die Reinheit werden keine Angaben gemacht.

Die Umsetzungen von CGC in flüssiger Phase liefern nach Abfiltrieren der Salze und einfacher destillativer Trennung vom Lösungsmittel und anderen Verunreinigungen ein rohes Vinylencarbonat, dass mit Resten Chloracetaldehyd, Chlorglykolcarbonat, Dichlorglykolcarbonat und weiteren zum Teil chlorhaltigen organischen Verbindungen verunreinigt ist.

Johnson und Patton beschrieben im JOC, 1960, S. 1042 die Umsetzung von CGC an festen Schüttungen von CaSO₄-Katalysatoren in der Gasphase bei 250°C und 6,67-8 hPa (50-60 Torr).

Die Katalysatoren deaktivieren sehr rasch und erreichen bestenfalls einem Umsatz von 35-40 %, bei einer Selektivität von 40-45 %. Höhere oder niedrigere Temperaturen führen zu weniger Umsatz. Die Katalysatoren können durch Abbrennen regeneriert werden.

Granulierte Aktivkohle und granuliertes aktiviertes Aluminiumoxid liefern nur gasförmige Produkte.

DE-A 1 135 452 beschreibt die HCl-Abspaltung von CGC bei 300-400°C. Das CGC wird gasförmig über ein inertes Trägermaterials geleitet, welches mit Elementen der I., II. oder VIII. Nebengruppe des Periodensystem bzw. deren Salzen oder Oxyden belegt ist. Vorzugsweise werden die Chloride des Eisens, Kobalts, Kupfers, besonders bevorzugt Cadmiumchlorid eingesetzt. Geeignete Trägermaterialien sind Bimse und Silicate mit Körnungen von 4 bis 8 mm.

Betrieben werden die Katalysatoren als stationäre Schüttung bei Normaldruck oder Unterdruck und einer Temperatur von 270 bis 450, bevorzugt von 300-400°C.

Das Verhalten von CdCl₂ auf Bimsstein wird explizit beschrieben. Der Katalysator ist mit ca. 270 Stunden deutlich standfester und mit 74 % selektiver als die CaSO₄-Kontakte

Die Belastung betrug 0,15 kg CGC pro L Katalysator und Stunde und der Inertgasstrom lag zwischen 27 bis 671 pro kg CGC. Der Durchschnittliche Umsatz betrug 87 %.

Der Katalysator kann bei 500 bis 700°C mit Luft abgebrannt werden.

Das Gasphasenverfahren zur Herstellung von Vinylencarbonat, liefert nach einer einfachen Destillation ein rohes Vinylencarbonat, dass bezüglich Verunreinigungen den Flüssigverfahren sehr ähnlich ist.

In Bezug auf den destillativen Reinigungsaufwand sind die Angaben in der Literatur ungenau, so dass man den Aufwand der im Einzelfall getrieben wurde und die Ausbeuteverluste durch die Reinigung nicht abschätzen kann.

Eine hohe Reinheit des VC besonders für die Anwendungen Polymerisation und Additiv für Batterieelektrolyte ist von hoher technischer Bedeutung.

In der US 2 873 230 wird beschrieben, dass VC nach der Methode von Newman und Addor hergestellt auch mit einer 80 bödigen Kolonne nicht ausreichend gereinigt werden kann, um mit Vinylacetat copolymerisiert zu werden und in der Homopolymerisation ungenügende Molekulargewichte erreicht werden.

Chlorhaltige Verunreinigungen werden dafür verantwortlich gemacht. Gegenstand der Anmeldung ist eine Reinigungsmethode die darin besteht, das feindestillierte VC zu verdampfen und gasförmig einer thermischen Behandlung bei 200 bis 450°C zuzuführen. Das so gewonnene VC wird nochmals feindestilliert, erst dann erreicht man eine Reinheit für befriedigende Polymerisationsergebnisse.

Huang et. al. beschreiben im Chin. J. Polm. Sci. (1990) 8 (3), 197-203, dass man VC hergestellt nach der Methode von Newman und Addor, nach dessen Isolierung durch Filtration und Abdestillieren des Lösungsmittels 1 Stunde mit ca. 4 % NaBH4 bei 64°C rührt und dann erst einer Feindestillation unterzieht. Diese Prozedur muss wiederholt werden, um verfärbungsstabiles gut polymerisierbares Material zu gewinnen.

Aus der JP2002/322171 ist ein Verfahren bekannt, bei dem man VC aus einem Toluol/Hexan-Lösungsmittel-Gemisch auskristallisieren lässt und dann nach 2-facher Waschung über eine Kolonne destilliert.

Die genannten Literaturstellen gehen nicht genau auf den Gehalt an Verunreinigungen ein, der im reinen VC verblieben ist. Verluste durch die Isolierungsprozedur sind ebenfalls offen.

Aufgabe der Erfindung ist die Bereitstellung eines Verfahrens zur destillativen Reinigung von Vinylencarbonat.

Überraschenderweise wurde gefunden, dass die gewünschten destillativen Eigenschafren von VC erreicht werden, wenn vor die Feindestillation eine einfache thermische Behandlung mit Harnstoff durchgeführt wird. Das gelingt, unabhängig davon, ob das technische VC durch Eliminierung in der flüssigen Phase oder in der Gasphase gewonnen wurde.

Gegenstand der Erfindung ist ein Verfahren zur Reinigung von Vinylencarbonat, bei dem das aufzureinigende Vinylencarbonat
a) bei einer Temperatur im Bereich von 25 bis 180°C mit einer organischen Verbindung mit mindestens einer amidischen Stickstoffwasserstoffbindung gegebenenfalls unter Zusatz von Lösungsmittel in Kontakt gebracht wird,
b) gegebenenfalls eventuell ausgefallener Feststoff abfiltriert wird,
c) und aus der verbliebenen Lösung über eine Kolonnendestillation das aufgereinigte Vinylcarbonat erhalten wird.

Die thermische Behandlung im Schritt a) erfolgt unter Rühren bei Temperaturen zwischen 25 und 180°C, bevorzugt zwischen 60 und 160°C, besonders bevorzugt zwischen 90 und 140°C.

Relativ zum Vinylencarbonat werden 0,1-30 Gew.-%, bevorzugt 1-10 Gew.-%, besonders bevorzugt 2-6 Gew.-% Harnstoff zugesetzt.

Der Zusatz von Harnstoff kann in Anwesenheit oder ohne Zusatz von Lösungsmitteln erfolgen. Als Lösungsmittel in Schritt a) können Dimethylacetamid, N-Methylpyrolidon (NMP), Dimethylformamid oder DMSO eingesetzt werden.

Nach der thermischen Behandlung wird gegebenenfalls eventuell ausgefallener Feststoff abfiltriert und das Vinylencarbonat vom Rückstand in Schritt c) abdestilliert. Dies kann als Batch-Destillation aus einem Behälter über eine Kolonne mit mindestens 10, bevorzugt mindestens 20, besonders bevorzugt mindestens 30 Böden geschehen.

Als Kolonneneinbauten sind alle dem Fachmann bekannten Möglichkeiten geeignet, z.B. Glockenböden, Siebböden, ferner Füllkörperschüttung wie z.B., Raschigringe, Pallringe, Berlsättel, außerdem Kreuzkanalstrukturen wie z.B. die Packungen der Firmen Sulzer und Montz.

Im Folgenden wird das erfindungsgemäße Verfahren anhand einiger Beispiele illustriert, wobei die Beispiele jedoch nicht als Einschränkung des Erfindungsgedankens zu verstehen sind.

### Beispiele:

Die Destillationsapparatur bestand aus einem ölbeheizten 15L-Planschliffiopf mit Ankerrührer, Kolonne, Rückflussteiler, Kondensator und einer Vorrichtung zum Einstellen eines konstanten Vakuums. Vor der Vakuumpumpe befand sich eine auf -78°C gekühlte Kühlfalle. Planschlifftopf, Kolonne, Rückflussteiler und Kondensator waren aus Glas, der Ankerrührer aus Teflon.

Die Kolonne hatte eine 1500 mm lange Sulzer DX Packung aus Hastelloy C mit einem Durchmesser von 50 mm. Packungen dieses Typs haben Trennleistungen zwischen 15 und 30 Böden pro Meter.

Die Apparatur wurde vor und nach dem Beschicken und vor dem Betrieb immer mit Stickstoff inertisiert.

Als Ausgangsstoff wurde durch eine Vordestillation ohne Kolonne im Wesentlichen lediglich von polymeren Verunreinigungen befreites rohes VC eingesetzt.

Dieses rohe VC war ca. 97 %ig und hatte einen Gehalt an organischem und anorganischem Chlor von ca. 0,5 % bis 1%.

Die gaschromatographische Analyse erfolgte mittel eines HP 6890. Getrennt wurde über eine 50 Meter lange CP-Sil 8 CB mit einem ID von 0,53 mm und einer FD von 1,0 µm.

Trägergas war Stickstoff mit einem Vordruck von 3,5 10⁴Pa (5 psi). Der Injektor wurde mit eine Flow von 138 ml/min und einem Split von 30/1 betrieben. Injiziert wurde 1 µL VC pur.

Die Injektortemperatur betrug 220°C, die Detektortemperatur 320°C. Das Temperaturprogramm startete mit 50°C, heizte mit 5°C/min bis 250°C auf.

Ausgewertet wurde nach der Norm%-Methode.

### Beispiel 1 (Vergleichsbeispiel)

7380 g dieses rohen VC wurden in der oben beschriebenen Apparatur bei 34-35 mbar zuerst unter vollständigem Reflux erhitzt und dann bei einem R/E von 50 Vorlauf abdestilliert. Bei einer Kopftemperatur von 62 bis 64°C destillierten in ca. 40 Stunden Fraktionen mit einem Gesamtgewicht von ca. 1460 g ab, die einen Gehalt von 88 bis 98 % VC und einen Chlorgehalt über 5000 ppm aufwiesen. Die Sumpftemperatur stieg dabei von 66 auf 70°C.

Bei 65 bis 67°C Kopftemperatur und einem Rücklaufverhältnis von 5/1 destillierten dann binnen 21 Stunden Fraktionen mit einem Gewicht von zusammen ca. 3700 g ab, die einen Gehalt an VC von ca. 99,5 % und einen Chlorgehalt von ca. 1000 ppm besaßen.

Die Sumpftemperatur stieg dabei von 70 auf 71°C, am Ende auf 76°C.

Anschließend destillierten in 10 Stunden 220 g VC mit einem Gehalt von ca. 99,2 % über, die einen Chlorgehalte von > 2000 ppm besaßen.

Die Sumpftemperatur stieg von 77 auf 84°C.

Am Ende blieben ca. 100g Sumpf übrig mit einem Gehalt von ca. 20 % VC.

In der Kühlfalle befanden sich ca. 400 g VC mit einem Gehalt von 37 %.

Die Massenbilanz betrug 92%, die VC-Bilanz ca. 78 %. 64 % des kondensierten Materials ist als 99,5 %iges VC gewonnen worden.

### Beispiel 2 (erfindungsgemäßes Beispiel)

12060 g rohes VC wurden mit 200 g Harnstoff versetzt und unter Stickstoff 2 Stunden bei 140°C gerührt. Nach Abkühlen auf ca. 30-40°C wurden 235 g Feststoff abfiltriert, 11743 g Flüssigkeit in die oben beschriebene Destillationsapparatur überfuhrt und mit 1000 g NMP versetzt.

Das Gemisch wurde bei ca. 35 mbar Druck zum Rückfluss erhitzt und anschließend mit einem Rücklaufverhältnis von 30/1 Vorlauf abdestilliert.

Innerhalb von 2,5 Stunden fielen so ca. 160 g Destillat an, dass laut GC-Analyse zu 96 % aus VC bestand. In den folgende 3,5 Stunden fielen ca. 400 g eines Destillates an, dass zu 97,5 % aus VC bestand, gefolgt von ca. 470 g Destillat mit 99,4 % VC Gehalt, die in 2,5 Stunden überdestillierten.

Nun wurde mit einem Rücklaufverhältnis von 5/1 der Hauptlauf abgenommen. In 26 Stunden destillierten ca. 9600 g eines 99,9 %igen VC über, dass einen Chlorgehalt unterhalb 50 ppm hatte.

Es blieben ca. 1100 g Sumpf zurück, mit einem VC-Gehalt von weniger als 0,5 %.

Die Kühlfalle war praktisch leer geblieben.

Die Massenbilanz war praktisch quantitativ, VC wurde zu 93 % wieder gefunden.
84 % des Vinylencarbonates sind in der Hauptfraktion angefallen.

## Patentansprüche

1. Verfahren zur Reinigung von Vinylencarbonat, bei dem das aufzureinigende Vinylencarbonat
a) bei einer Temperatur im Bereich von 25 bis 180°C mit 0,1 bis 30 Gew.-% Harnstoff, bezogen auf das Vinylencarbonat, gegebenenfalls unter Zusatz von Lösungsmittel in Kontakt gebracht wird,
b) eventuell ausgefallener Feststoff abfiltriert wird,
c) und aus der verbliebenen Lösung über eine Kolonnendestillation das aufgereinigte Vinylencarbonat erhalten wird.

2. Verfahren nach Anspruch 1, **dadurch gekennzeichnet, dass** als Lösungsmittel in Schritt a) Dimethylacetamid, N-Methylpyrolidon, Dimethylformamid oder DMSO zugesetzt wird.

## Claims

1. Process for the purification of vinylene carbonate, in which the vinylene carbonate to be purified
a) is brought into contact at a temperature in the range 25 to 180°C with 0.1% to 30% by weight of urea, based on the vinylene carbonate, optionally with addition of solvent,
b) any precipitated solid is filtered off,
c) and the purified vinylene carbonate is obtained from the remaining solution via a column distillation.

2. Process according to Claim 1, **characterized in that** dimethylacetamide, N-methylpyrrolidone, dimethylformamide or DMSO is added as a solvent in step a).

## Revendications

1. Procédé de purification de carbonate de vinylène, selon lequel le carbonate de vinylène à purifier
a) est mis en contact à une température dans la plage allant de 25 à 180 °C avec 0,1 à 30 % en poids d'urée, par rapport au carbonate de vinylène, éventuellement avec ajout d'un solvant,
b) un solide précipité est éventuellement éliminé par filtration,
c) et le carbonate de vinylène purifié est obtenu à partir de la solution restante par une distillation en colonne.

2. Procédé selon la revendication 1, **caractérisé en ce que** du diméthylacétamide, de la N-méthylpyrrolidone, du diméthylformamide ou du DMSO est ajouté à l'étape a) en tant que solvant.
